# EUROPEAN PATENT SPECIFICATION

(11) **EP 0 521 225 B1**
(45) Date of publication and mention of the grant of the patent: **12.10.1994**
(21) Application number: 91810523.0
(22) Date of filing: 04.07.1991
(51) Int. Cl.: A61K 31/42, A61K 31/43, A61K 31/545, A61K 31/415, A61K 31/155, A61K 31/185, A61K 31/54

(54) **Pharmaceutical preparation for treatment of periodontitis, containing clavulanic acid**
Pharmazeutische Zubereitungen, Clavulansäure enthaltend, für die Behandlung der Periodontitis
Compositions pharmaceutiques pour le traitement de la parodontose, contenant l'acide clavulanique

(43) Date of publication of application: 07.01.1993
(73) Proprietor: Hawe-Neos Dental Dr. H. v. Weissenfluh SA, 6934 Bioggio (CH)
(72) Inventor: Mühlemann, Hans R., Prof.Dr., CH-8702 Zollikon (CH)
(74) Representative: AMMANN PATENTANWAELTE AG BERN

(56) References cited:
- WO-A-86/07265
- FR-A- 2 309 231
- FR-A- 2 316 954
- US-A- 4 919 939
- MINERVA STOMATOL, vol. 37, no. 12, December 1988, pages 1005-1009; G.C. ZOCCOLAet al.: "Valutazione clinica dell'efficacia terapeutica dell'associazioneamoxicillina-acido clavulanico nella patologia odontostomatologica acuta"
- ANTIMICROB. AGENTS CHEMOTHER., vol. 34, no. 8, August 1990, pages 1546-1550; P.C. APPELBAUM et al.: "Beta-lactamase production and susceptibilities toamoxicillin, amoxicillin-clavulanate, ticarcillin, ticarcillin-clavulanate,cefoxitin, imipenem, and metronidazole of 320 non-bacteroides fragilisbacteroides isolates 129 fusobacteria from 28 U.S. centers"
- J. DENT., vol. 19, no. 2, April 1991, pages 92-96; S.H. FANAS-ABU et al.:"Evaluation of acrylic strips containing amoxycillin with clavulanic acid forlocal drug delivery"
- J.E.F. REYNOLDS et al.: "Martindale - the extra pharmacopoeia", 28th edition,1982, pages 1143-1144, Pharmaceutical Press, London, GB
- ANTIMICROB. AGENTS CHEMOTHER., vol. 35, no. 5, May 1991, pages 886-891; J.M.LACROIX: "Characterization of a beta-lactamase found in Eikenella corrodens"
- J. ANTIMICROB. CHEMOTHER., vol. 26, no. 3, September 1990, pages 353-359; K.E.ALDRIDGE: "In-vitro study of the susceptibility of cefoxitin/cefotetanresistant bacteroides fragilis group strains to various other antimicrobialagents"
- HELVETICA ODONTOLOGICA ACTA, vol. 19, no. 2, 27th February 1976, pages 57-60,ZÜRICH, CH; H.R. MÜHLEMANN et al.: "Inhibition of plaque growth with taurolin,vantocil and amine fluoride"
- PHARMATHERAPEUTICA, vol. 2, no. 8, 1981, pages 517-522; M. KENNEDY BROWNE: "Thetreatment of peritonitis by an antiseptic taurolin"

## Description

One widespread reason for the loss of teeth is periodontitis, a destructive disease. Under healthy conditions, by means of a specialized epithelial collar, an attachment is formed between a sulcular gingival soft tissue 1 and the calcified structure of the tooth 3. Periodontitis is initiated by gingivitis, an inflammatory reaction of the marginal gingivae 2 and the sulcus areas 1. Gingivitis and periodontitis are caused by bacterial accumulations. They are "dental plaque-diseases".

Initial gingivitis is mainly associated with mutans streptococci causing inflammation of the superficial marginal gingivae 2. Gingivitis can be treated successfully.

Gingivitis, under poor dental hygienic conditions, may progress into periodontitis which also is inflammatory, often suppurating (pyorrhea) leading to the destruction of the subgingival tooth supporting structures. The periodontal infection destroys:
1) the connective tissue of the periodontal membrane 5,
2) the tooth supporting periodontal bone 6.

The progressive, generally irreversible damage results in periodontal pockets 4 which are lined by and filled with a Gram-negative periodontal plaque flora.

The treatment of subgingival plaque is difficult. There are various approaches to reduce the periodontapathic pocket bacteria. Local procedures are mechanical and/or chemical in nature.

Surgical removal of infected pockets has been successfully achieved -- but in single cases only.

The mechanical periodontal pocket debridement (curettage) and cleaning of the pocket walls by dental professionals is quite successful but tedious, time consuming, and costly. Because of bacterial re-immigration it has to be performed 3 to 6 times a year, depending on the quality of individual oral hygiene.

The primary object of pocket disinfection methods is the complete and permanent removal of all pocket microorganisms. Various systemic antibiotics and chemotherapeutics have been essayed. A comprehensive review of antibiotics in systemic therapy has been presented by Slots and Rams, J. Clin. Periodontol. 1990, pp. 479-493. Reduction of chronic periodontal infections and inflammations were transitory. In general, systemic antibacterials are most efficient for short term treatments and acute infections. They have not solved the periodontal pocket problem.

Local disinfection of subgingival pockets has been investigated thoroughly: Oral mouth rinses with various antibacterials were disappointing. Local irrigations of pockets with irrigators, sprays, or syringes suffered from the rapid clearance of the aqueous disinfectants. Pocket-irrigations with antibiotic gels had slightly longer clearance times.

A further problem of infected periodontal pockets relates to the endotoxins set free when Gram-negative bacteria are inactivated. Endotoxins interfere in the pocket with the healing attempts of surrounding fibroblasts.

Another approach consists in using sustained release systems which topically release a pharmaceutical agent over longer time periods into their environment. The sustained release systems generally belong to the field of topical medication and comprise a carrier in which the drug is embedded or contained. One such system used also in studies about periodontitis (S. H. Abu Fanas et. al, J. Dent 19 (1991) 92 - 96) uses acrylic strips placed in the pocket and containing a therapeutically effective agent, e.g. an antibiotic, which slowly diffuses out of the strips. However, there are besides others the following drawbacks in the application of those strips:
a) the periodontal pocket is not closed to the buccal cavity, so the agents are washed out of the pocket and new bacteria can easily immigrate;
b) the patient has to take great care in eating and drinking to not inadvertantly push the acrylic strips deeper in the pocket leading to lesions of the gingiva, or to remove the strips from the pocket, and
c) the acrylic strips have to be manually removed by the dentist after use.

US-patent US-A-4,780,320, US-patent US-A-4,919,939, and the European patent application EP-A1-0,244,118 disclose a method of controlled release of a drug in the periodontal pocket over time periods up to 30 days. A preferred embodiment of the system consists of microparticles containing the drug and a pharmaceutical acceptable carrier which is fluid at room temperature and gels at body temperature, i.e. after application of the preparation into the periodontal pocket. For example, PLURONIC (TM) F127 of BASF Wyandotte may be used as gelling carrier. The fluid carrier allows the application by e. g. a syringe, while the hardening ensures that the preparation remains in the pocket without further precautions by the patient. The drug is released by the microparticles with a rate given by the chemical and physical properties of the drug, the material of the microparticles, and the surrounding carrier. In a further embodiment, the carrier as well as the microparticles are biologically degradable, whereby the necessity to remove the worn preparation out of the periodontal pocket is avoided.

However, from a pharmaceutical perspective, the effectivity of the used drug is the crucial point. As the drug is released at a rate which varies within certain ranges, and as the wearing time of the drug should be as long as possible, it is important to consider the effectivity of the used drug. With longer stand time of the preparation in the periodontal pocket, the rate of release must be reduced. In consequence, the concentration of the drug in the gingival fluid of the periodontal pocket is lowered and may be too small to be effective. It is astonishing, that none of the patents concerned with the controlled release system refers to the effectivity of the treatment. Furtheron, the used drugs are only included because of their antibiotic efficiency, and the specifications describe only the use of one single antibacterial substance in a preparation.

Recently, new antibiotic preparations have been developed comprising β-lactamase inhibitors, e. g. clavulanic acid. The β-lactamase inhibitors inhibit the degradation of the β-lactams by the β-lactamase. In addition, they themselves are antibiotic agents. The β-lactams comprise the most used antibiotics: penames (penicilline), ceph-3-ems (cephalosporin), clavams (clavulanic acid), carbapenemes and mono-lactames. Preparations containing β-lactamase inhibitors are known for systemic or oral applications. A mixture of amoxicillin, an antibiotic of the penicillin-type, and clavulanic acid has been contemplated (S. H. Abu Fanas et al., J. Dent. 19 (1991) 92 - 96). The mixture has been embedded in acrylic strips as described above and the strips introduced in the periodontal pockets. The effectivity of this agent combination has been found equivalent to that of tetracycline, another known antibiotic also effective against Gram-negative bacteria.

The influence of the endotoxin with respect to periodontitis has been investigated (e.g. B. I. Simon, H. M. Goldman, M. P. Ruben, and E. Baker, J. Periodontol. 41 (1970) 81-86; ibid. 42 (1971) 210-216; ibid. 43 (1972) 468-475). In general, anti-endotoxin agents are well known. One broadly applicable agent is taurolidine and its derivatives. However, the anti-endotoxin agents are used preferably in the field of treatment of peritonitis, pancreatitis and osteotitis wherein also the antibiotic effect is used. The application in the field of dental medication has been tried, but did not lead to superior results in comparision with known antibiotics (S. Reynolds, W. Wade, M. Addy, and J. Moran, The In-vitro Antibacterial Activity of Taurolin(R), University of Wales Dental School, Heat Park, Cardiff, S. Wales).

Further investigations concerning the effectivity of taurolidine or derivatives thereof as sole effective agent have been performed in the field of dentistry: French patent application publications FR-A-2 316 954 and FR-A-2 309 231, and H.R. Mühlemann and J.R. Straub, Helv. Odont. Acta 19/2 (1975) 57 - 60. The forms of application in these investigations have been mouth-rinses, tooth pastes and the like. One known use of taurolidine in a release system for topic application provides a material for repairing bone damages wherein the material contains agents to promote the healing process. This material, described in the patent application publication WO-A-86/07265, is integrated in the bone during the healing process. As the antibiotic and anti-endotoxic agent, taurolidine is proposed. The carrier consisted of specially prepared natural bone material.

However, all the known pharmaceutical preparations used in sustained release systems did not perform significantly better as three problems posed by periodontits are not equally respected: 1.: Combat of the mostly Gram-negative bacteria in the periodontal pocket; 2.: Suppression of the effects of the residues of the bacteria, the endotoxins; 3.: Inhibition of reimmigration of bacteria into the pocket.

Therefore, in the field of therapy and prophylaxis of periodontitis, it is one object of the present invention to provide a pharmaceutical preparation of improved effectivity for topical application in the periodontal pocket.

It is another object of the invention to accelerate the healing process with which the bacterial endotoxin interferes.

These objects are attained by the pharmaceutical preparation as defined by the claims.

An important progress in the treatment of periodontitis is the use of a system of controlled release of a drug into the periodontal pocket according to US-A-4,780,320 or US-A-4,919,939. Preferably, this controlled release system involves a carrier which gels in the pocket whereby the pocket is closed with respect to the mouth. Furthermore, the medication sustains a rather constant concentration of the included drug in the pocket over periods up to one month.

It has been found that the antibiotic effect is improved by suppressing the effect of β-lactamase by β-lactamase inhibitors. Often, β-lactamase inhibitors used alone or as synergistic additives to β-lactam antibiotics can attack bacteria which are otherwise immune. The β-lactamase inhibitors are effective especially in the treatment of diseases in which Gram-negative bacteria are involved. It has been found that, for example, such compounds are the clavams, more specially the clavulanic acid.

The effectivity of the usual antibiotic compound, for example penicilline or ampicillin, may be strengthened by the addition of a β-lactamase inhibitor whereby often additive or synergistic effects are obtained. Especially, the antibiotic compound is shielded against the attack of the β-lactamase and therefore is effective also against bacteria which were otherwise immune. However, the combination of a β-lactamase inhibitor and an antibiotic for use in a controlled release drug system is problematic because the rate of release of the two components must be correlated whereby stringent requirements are imposed on the release system. Surprisingly, this is no problem with the above mentioned system of the invention, and the effectivity of the released combination of antibiotic agent and β-lactamase inhibitor is remarkably increased.

Any of the known antibiotics may be chosen as the antibacterial agent, for example, it may be penicillin, amoxycillin, ampicillin, cephalosporine, etc. of the β-lactam group. However, the range of applicable antibiotics is not limited to the aforegoing enumeration.

Besides antibiotic measures, the healing process, especially the new attachment of the gingiva to the tooth socket, has been considered. Bacterial endotoxin impairs the attachment of fibroblasts to dental collagen and also to hard dental surfaces. Therefore it has been contemplated that it may be favourable to include also an anti-endotoxin component, e. g. taurolidine. The anti-endotoxin agent accelerates healing processes significantly. Surprisingly, the further addition of this agent to the preparation for use in the controlled release system also does not give rise to problems.

The preparations as described above replace preferably the single drug in the controlled release system as disclosed e.g. in the EP-A-0,244,118 and lead to superior pharmaceutical efficiency.

For the inclusion of the agent or the agents in a controlled release system, they may be dissolved, emulsified or suspended by adding them in arbitrary sequence to a liquid phase in an adequate mixing device according to the process described in the US-patent US-A-4,780,320. Alternatively, the agents may be added as a ready mixture which may be prepared by mixing the pure agents in an appropriate mixing device temperature, mixing forces etc. being observed to be non-destructive with regard to the agents. Preferably, the mixing is performed at room temperature under mild conditions. The examples below are carried out using one of these methods of inclusion of the agent or the agents.

The invention is further explained by way of examples which do not limit the scope of the invention.

### Example 1

The pharmaceutical preparation consists of ampicillin as antibiotic, and clavulanic acid as the β-lactamase inhibitor. The pharmaceutical preparation is enclosed in microparticles of a polyanhydride, poly(bis(p-carboxyphenoxy)methane), and the microparticles are suspended in PLURONIC (TM) F127 of BASF Wyandotte according to the US-A-4, 919,939, Example 12. The suspension may be applied to the periodontal pocket by a syringe. After application, the carrier as well as the microparticles will be superficially attacked and degraded whereby the pharmaceutical preparation is released into the pocket.

### Example 2

Example 1 was repeated the preparation further comprising taurolidine as an anti-endotoxin agent.

### Example 3

Example 1 was repeated with the exception that the antibiotic agent was replaced by taurolidine as an anti-endotoxin agent. Taurolidine as well as clavulanic acid are themselves antibiotics, therefore the specialized antibiotic agent is not always necessary.

Also, the healing process may be promoted by use of this preparation as a second measure after having exclusively destroyed the bacteria by application of a preparation according to example 1. Instead of the erosive release system, it also possible to use any of the release systems as disclosed in the document US-A-4,919,939.

The agents of the preparations as described in the three Examples above are released over about one week or more.

Of course, the pharmaceutical preparation as disclosed by the present invention may be used as part of any release system for treatment of periodontitis or other local inflammations caused directly or indirectly by bacteria. The preparation may further contain anti-inflammatory agents and/or growth promoters.

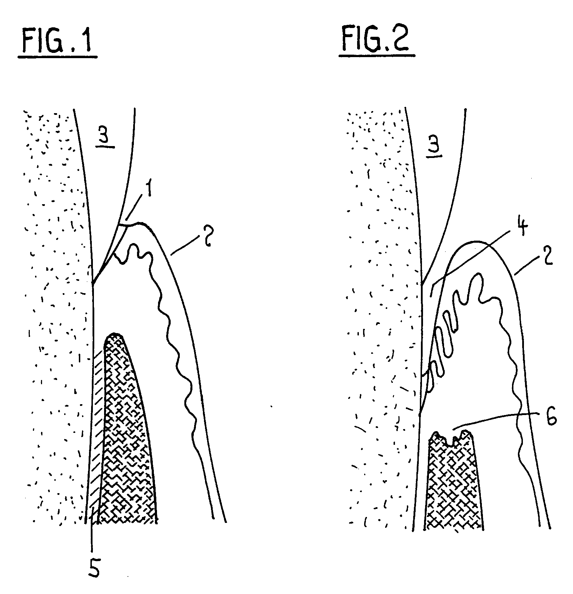

## Claims

1. A pharmaceutical preparation for topical therapy and prophylaxis of periodontitis within the periodontal pocket, characterized in that the preparation comprises at least one β-lactamase inhibitor, at least one anti-endotoxin agent, and that the preparation is contained in a controlled release system.

2. A pharmaceutical preparation according to claim 1, characterized in that the β-lactamase inhibitor is a clavam.

3. A pharmaceutical preparation according to one of claims 1 or 2, characterized in that the β-lactamase inhibitor is clavulanic acid.

4. A pharmaceutical preparation according to any one of claims 1 to 3, characterized in that one of the anti-endotoxin agents is a derivative of taurine.

5. A pharmaceutical preparation according to claim 4, characterized in that one of the anti-endotoxin agents is taurolidine.

6. A pharmaceutical preparation according to any one of claims 1 to 5, characterized in that it further comprises at least one antibiotic agent.

7. A pharmaceutical preparation according to claim 6, characterized in that the antibiotic is a β-lactam.

8. A pharmaceutical preparation according to claim 7 characterized in that the antibiotics are chosen from the antibiotic compounds of the substance classes penicillin, cephalosporin, carbapenem, or monolactam.

9. A pharmaceutical preparation according to claim 8, characterized in that at least one of the antibiotics is ampicillin, amoxycillin, metronidazole, or chlorhexidine.

10. A pharmaceutical preparation according to any one of claims 1 to 9, characterized in that the controlled release system comprises microparticles and a pharmaceutically acceptable carrier.

11. A pharmaceutical preparation according to claim 10, characterized in that the carrier of the controlled release system is fluid before application and gels or hardens under the conditions of the place of application.

## Patentansprüche

1. Pharmazeutische Zubereitung zur örtlichen Behandlung und Prophylaxe von Periodontitis in der Periodontaltasche, dadurch gekennzeichnet, dass die Zubereitung mindestens einen β-Lactamase-Inhibitor und mindestens ein Antiendotoxin enthält, und dass die Zubereitung in einem System zur geregelten Abgabe enthalten ist.

2. Pharmazeutische Zubereitung nach Anspruch 1, dadurch gekennzeichnet, dass der β-Lactamase-Inhibitor ein Clavam ist.

3. Pharmazeutische Zubereitung nach einem der Ansprüche 1 oder 2, dadurch gekennzeichnet, dass der β-Lactamase-Inhibitor Clavulansäure ist.

4. Pharmazeutische Zubereitung nach einem der Ansprüche 1 bis 3, dadurch gekennzeichnet, dass eines der Antiendotoxine ein Taurinderivat ist.

5. Pharmazeutische Zubereitung nach Anspruch 4, dadurch gekennzeichnet, dass eines der Antiendotoxine Taurolidin ist.

6. Pharmazeutische Zubereitung nach einem der Ansprüche 1 bis 5, dadurch gekennzeichnet, dass sie weiterhin mindestens ein Antibioticum enthält.

7. Pharmazeutische Zubereitung nach Anspruch 6, dadurch gekennzeichnet, dass das Antibioticum ein β-Lactam ist.

8. Pharmazeutische Zubereitung nach Anspruch 7, dadurch gekennzeichnet, dass die Antibiotica aus antibiotischen Verbindungen der Substanzklassen Penicillin, Cephalosporin, Carbapenem oder Monolactam ausgewählt sind.

9. Pharmazeutische Zubereitung nach Anspruch 8, dadurch gekennzeichnet, dass mindestens eines der Antibiotica Ampicillin, Amoxycillin, Metronidazol oder Chlorhexidin ist.

10. Pharmazeutische Zubereitung nach einem der Ansprüche 1 bis 9, dadurch gekennzeichnet, dass das System mit geregelter Abgabe Mikroteilchen und einen pharmazeutisch zulässigen Trägerstoff enthält.

11. Pharmazeutische Zubereitung nach Anspruch 10, dadurch gekennzeichnet, dass der Trägerstoff des Systems mit geregelter Freigabe vor der Anwendung flüssig ist und unter den Bedingungen am Ort der Anwendung geliert oder härtet.

## Revendications

1. Composition pharmaceutique pour la thérapie et la prophylaxie topiques de la parodontose dans l'intérieur de la poche parodontale, caractérisée en ce que la composition contient au moins un inhibiteur de β-lactamase et au moins un agent anti-endotoxine, et que la composition est comprise dans un système de libération contrôlée.

2. Composition pharmaceutique selon la revendication 1, caractérisée en ce que l'inhibiteur de β-lactamase est un clavame.

3. Composition pharmaceutique selon l'une des revendications 1 ou 2, caractérisée en ce que l'inhibiteur de β-lactamase est l'acide clavulanique.

4. Composition pharmaceutique selon l'une quelconque des revendications 1 à 3, caractérisée en ce qu'un des agents anti-endotoxines est un dérivé de taurine.

5. Composition pharmaceutique selon la revendication 4, caractérisée en ce qu'un des agents anti-endotoxines est la taurolidine.

6. Composition pharmaceutique selon l'une quelconque des revendications 1 à 5, caractérisée en ce qu'elle contient en plus au moins un agent antibiotique.

7. Composition pharmaceutique selon la revendication 6, caractérisée en ce que l'antibiotique est un β-lactame.

8. Composition pharmaceutique selon la revendication 7, caractérisée en ce que les antibiotiques sont choisis parmi les composés antibiotiques de la classe des substances pénicilline, céphalosporine, carbapénème ou monolactame.

9. Composition pharmaceutique selon la revendication 8, caractérisée en ce qu'au moins un des antibiotiques est l'ampicilline, l'amoxycilline, le métronidazole ou le chlorohexidine.

10. Composition pharmaceutique selon l'une quelconque des revendications 1 à 9, caractérisée en ce que le système à libération contrôlée contient des microparticules et un véhicule pharmaceutiquement acceptable.

11. Composition pharmaceutique selon la revendication 10, caractérisée en ce que le véhicule du système à libération contrôlée est liquide avant l'application et forme un gel ou durcit dans les conditions existant à l'endroit de l'application.
